# EUROPEAN PATENT APPLICATION

(11) **EP 2 353 578 A2**
(43) Date of publication of application: **10.08.2011**
(21) Application number: 11250128.3
(22) Date of filing: 04.02.2011
(51) Int. Cl.: A61K 8/19, A61K 8/27, A61Q 19/00

(54) **Lip compositions comprising galvanic particulates**

(30) Priority: 05.02.2010 US 301944 P
(71) Applicant: Johnson & Johnson Consumer Companies, Inc., Skillman, NJ 08558 (US)
(72) Inventor: Brunning, Elizabeth, Somerset, NJ 08873 (US); Chantalat, Jeannette, Pennington, NJ 08534 (US); Maitra, Prithwiraj, Randolph, NJ 07869 (US)
(74) Representative: Kirsch, Susan Edith

(57) **Abstract**

Novel lip compositions are disclosed. The lip compositions comprise galvanic particulates and provide the following benefits: enhanced lip color, reduced fine lines and wrinkles, increased fullness, improved moisturization, smoothness, texture, and improved definition and lip contour.

## Description

The present invention relates to new lip compositions, such as lipsticks, lip glosses, lip balms, and lip pencils, comprising galvanic particulates.

### BACKGROUND OF THE INVENTION

Topical compositions comprising galvanic particulates are described in WO 2009/045720 and US 2007/0060862, which indicate that a variety of skin benefits may be achieved therefrom. For example, WO 2009/045720 discloses that galvanic particulates may increase soft tissue volume by increasing collagen or elastin in the skin or lips.

Lip compositions are widely used cosmetic products. They are typically intended to provide color or texture to the lips. Lip balms also may have a medicinal component.

It has now been discovered that lip compositions may be formulated with galvanic particulates to provide the specific benefits of enhanced lip color, reduced fine lines and wrinkles, increased fullness, improved moisturization, smoothness, texture, and improved definition and lip contour.

Applicants have also discovered that topical application of a lip composition comprising galvanic particulates to the lips increases oxy-hemoglobin levels in the lips by at least 10 percent.

### SUMMARY OF THE INVENTION

The invention provides a lip composition comprising: a) at least one structuring agent selected from thickeners, fatty substances, and waxes, and b) galvanic particulates comprising a first conductive material and a second conductive material, wherein both said first conductive material and said second conductive material are exposed on the surface of said galvanic particulates, the particle size of said galvanic particulates is from about 10 nanometers to about 100 micrometers, and the difference in Standard Potentials of the first conductive material and the second conductive material is at least about 0.2 V.

The invention also provides a method of increasing oxy-hemoglobin levels in lips by at least 10 percent, comprising topically applying to the lips a lip composition comprising galvanic particulates comprising a first conductive material and a second conductive material, wherein both said first conductive material and said second conductive material are exposed on the surface of said galvanic particulates, the particle size of said galvanic particulates is from about 10 nanometers to about 100 micrometers, and the difference in Standard Potentials of the first conductive material and the second conductive material is at least about 0.2 V.

### BRIEF DESCRIPTION OF THE DRAWINGS

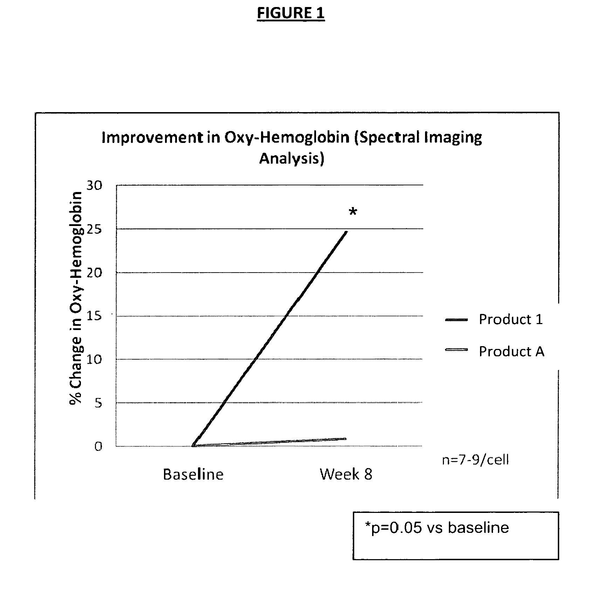

Figure 1 depicts the results of the oxy-hemoglobin analysis done on the spectral images described in Example 2.

### DETAILED DESCRIPTION OF THE INVENTION

Unless defined otherwise, all technical and scientific terms used herein have the same meaning as commonly understood by one of ordinary skill in the art to which the invention pertains. Also, all publications, patent applications, patents, and other references mentioned herein are incorporated by reference. Unless otherwise indicated, a percentage refers to a percentage by weight (i.e., %(W/W)).

As used herein, "cosmetically-acceptable" means suitable for use in contact with tissues (e.g., the skin) without undue toxicity, incompatibility, instability, irritation, allergic response, or the like. This term is not intended to limit the composition it describes as for use solely as a cosmetic (e.g., the composition may be used as a pharmaceutical).

As used herein, "safe and effective amount" means an amount sufficient to provide a desired benefit at a desired level, but low enough to avoid serious side effects.

As used herein, the term "treating" or "treatment" means alleviation or elimination of symptoms, cure, prevention, or inhibition of a human condition or disease, specifically of the lips.

The present invention relates to a lip composition, such as a lipstick, lipcare product, lip pencil or liner, lip scrub, or lip gloss. The lip composition may take any one of a variety of forms, including liquid forms, hot pour sticks, molded sticks, or gel sticks. The lip composition may be colored or uncolored, clear, translucent or opaque, and may be used for cosmetic purposes or therapeutic purposes.

The lip composition comprises galvanic particulates. Each galvanic particulate comprises a first conductive material and a second conductive material, wherein both the first conductive material and the second conductive material are exposed on the surface of the galvanic particulate. In one embodiment, the galvanic particulates comprise the first conductive material partially coated with the second conductive material.

Preferably, the lip composition comprises up to about 10 weight percent galvanic particulates, for example up to about 5 weight percent galvanic particulates or up to about 1 weight percent galvanic particulates.

The galvanic particulates may be produced by a coating method wherein the weight percentage of the second conductive material is from about 0.001 % to about 20%, by weight, of the total weight of the particulate, such as from about 0.01 % to about 10%, by weight, of the total weight of galvanic particulate. The coating thickness of the second conductive material may vary from single atom up to hundreds of microns. The surface of the galvanic particulate may comprise from about 0.001 percent to about 99.99 percent such as from about 0.1 to about 99.9 percent of the second conductive material.

The galvanic particulates are produced by a non-coating method (e.g., by sintering, printing or mechanical processing the first and the second conductive materials together to form the galvanic particulate) wherein the second conductive material comprises from about 0.1 % to about 99.9%, by weight, of the total weight of the particulate, such as from about 10% to about 90%, of the total weight of the particulate.

The galvanic particulates are preferably fine enough that they can be suspended in semi-solid compositions during storage. The galvanic particulates maybe flattened and/or elongated shapes. The advantages of flattened and elongated shapes of the galvanic particulates include a lower apparent density and, therefore, a better floating/suspending capability in the lip composition, as well as better coverage over the lips, leading to a wider and/or deeper range of the galvanic current passing through the lips. In one embodiment, the longest dimension of the galvanic particulates is at least twice (e.g., at least five times) the shortest dimension of such particulates.

The galvanic particulates may be of any shape, including but not limited to, spherical or non-spherical particles or elongated or flattened shapes (e.g., cylindrical, fibers or flakes). The average particle size of the galvanic particulates is from about 10 nanometers to about 500 micrometers, such as from about 100 nanometers to about 100 micrometers. What is meant by the particle size is the maximum dimension in at least one direction.

The galvanic particulate may comprise at least 90 percent by weight of conductive materials (e.g., the first conductive material and the second conductive material), such as at least 95 percent by weight, or at least 99 percent by weight, when a coating method is used for the production of the galvanic particulates.

Examples of combinations of first conductive materials/second conductive materials include (with a "/" sign representing an oxidized but essentially non-soluble form of the metal), but are not limited to, zinc-copper, zinc-copper/copper halide, zinc-copper/copper oxide, magnesium-copper, magnesium-copper/copper halide, zinc-silver, zinc-silver/silver oxide, zinc-silver/silver halide, zinc-silver/silver chloride, zinc-silver/silver bromide, zinc-silver/silver iodide, zinc-silver/silver fluoride, zinc-gold, zinc-carbon, magnesium-gold, magnesium-silver, magnesium-silver/silver oxide, magnesium-silver/silver halide, magnesium-silver/silver chloride, magnesium-silver/silver bromide, magnesium-silver/silver iodide, magnesium-silver/silver fluoride, magnesium-carbon, aluminum-copper, aluminum-gold, aluminum-silver, aluminum-silver/silver oxide, aluminum-silver/silver halide, aluminum-silver/silver chloride, aluminum-silver/silver bromide, aluminum-silver/silver iodide, aluminum-silver/silver fluoride, aluminum-carbon, copper-silver/silver halide, copper-silver/silver chloride, copper-silver/silver bromide, copper-silver/silver iodide, copper-silver/silver fluoride, iron-copper, iron-copper/copper oxide, copper-carbon iron-copper/copper halide, iron-silver, iron-silver/silver oxide, iron-silver/silver halide, iron-silver/silver chloride, iron-silver/silver bromide, iron-silver/silver iodide, iron-silver/silver fluoride, iron-gold, iron-conductive carbon, zinc-conductive carbon, copper-conductive carbon, magnesium-conductive carbon, and aluminum-carbon.

The first conductive material or second conductive material may also be an alloy, particularly the first conductive material. Non-limiting examples of alloys include alloys of zinc, iron, aluminum, magnesium, copper and manganese as the first conductive material and alloys of silver, copper, stainless steel and gold as second conductive material.

The galvanic particulate may comprise the first conductive material partially coated with several conductive materials, such as with a second and third conductive material. The particulate may comprise at least 95 percent, by weight, of the first conductive material, the second conductive material, and the third conductive material. In one embodiment, the first conductive material is zinc, the second conductive material is copper, and the third conductive material is silver.

The difference in the Standard Electrode Potentials (or simply, Standard Potential) of the first conductive material and the second conductive material may be at least about 0.1 volts, such as at least 0.2 volts. In one embodiment, the materials that make up the galvanic couple have a Standard Potential difference equal to or less than about 3 volts. For example, for a galvanic couple comprised of metallic zinc and copper, the Standard Potential of zinc is -0.763V (Zn/Zn2⁺), and the Standard Potential of copper is +0.337 (Cu/Cu2⁺), the difference of the Standard Potential is therefore 1.100V for the zinc-copper galvanic couple. Similarly, for the magnesium-copper galvanic couple, Standard Potential of magnesium (Mg/Mg2⁺) is -2.363V, and the difference of the Standard Potential is therefore 2.700V. Additional examples of Standard Potential values of some materials suitable for use in galvanic particulates are: Ag/Ag⁺: +0.799V, Ag/AgCl/Cl⁻: 0.222V, and Pt/H₂/H⁺: 0.000V. Pt may also be replaced by carbon or another conductive material. See, *e.g.,* Physical Chemistry by Gordon M. Barrow, 4th Ed., McGraw-Hill Book Company, 1979, page 626.

In one embodiment, the first and second conductive electrodes are combined (e.g., the second conductive electrode is deposited to the first conductive electrode) by chemical, electrochemical, physical or mechanical process (such as electroless deposition, electric plating, vacuum vapor deposition, arc spray, sintering, compacting, pressing, extrusion, printing, and granulation) conductive metal ink (e.g., with polymeric binders), or other known metal coating or powder processing methods commonly used in powder metallurgy, electronics or medical device manufacturing processes, such as the methods described in the book Asm Handbook Volume 7: Powder Metal Technologies and Applications (Asm International Handbook Committee, edited by Peter W. Lee, 1998, pages 31-109, 311-320). In another embodiment, all the conductive electrodes are manufactured by chemical reduction processes (e.g., electroless deposition), sequentially or simultaneously, in the presence of reducing agent(s). Examples of reducing agents include phosphorous-containing reducing agents (e.g., a hypophosphite as described in US Patent Nos 4,167,416 and 5,304,403), boron-containing reducing agents, and aldehyde- or keton-containing reducing agents such as sodium tetrahydridoborate (NaBH₄) (*e.g*., as described in US 20050175649).

In one embodiment, the second conductive electrode is deposited or coated onto the first conductive electrode by physical deposition, such as spray coating, plasma coating, conductive ink coating, screen printing, dip coating, metals bonding, bombarding particulates under high pressure-high temperature, fluid bed processing, or vacuum deposition.

In one embodiment, the coating method is based on displacement chemical reaction, namely, contacting particles of the first conductive material (e.g., metallic zinc particles) with a solution containing a dissolved salt of the second conductive material (e.g. copper acetate, copper lactate, copper gluconate, or silver nitrate). In a further embodiment, the method includes flowing the solution over particles of the first conductive material (e.g., zinc powder) or through a packed powder of the first conductive material. In one embodiment, the salt solution is an aqueous solution. In another embodiment, the solution is contains an organic solvent, such as an alcohol, a glycol, glycerin or other commonly used solvents in pharmaceutical production to regulate the deposition rate of the second conductive material onto the surfaces of the first conductive material particles, therefore controlling the activity of the galvanic particulates produced.

The galvanic particulates of the present invention may also be coated with other materials to protect the first and second conductive materials from degradation during storage (e.g., oxidation degradation from oxygen and moisture), or to modulate the electrochemical reactions and to control the electric current generated when in use. Exemplary coating materials include inorganic or organic polymers, natural or synthetic polymers, biodegradable or bioabsorbable polymers, silica, glass, various metal oxides (e.g., oxide of zinc, aluminum, magnesium, or titanium) and other inorganic salts of low solubility (e.g., zinc phosphate). Coating methods are known in the art of metallic powder processing and metal pigment productions, such as those described in US 5,964,936; U.S. 5,993,526; US 7,172,812; US 20060042509A1 and US 20070172438.

In one embodiment, the galvanic particulates are stored in anhydrous form, e.g., as a dry powder or as an essentially anhydrous non-conducting organic solvent composition (e.g., dissolved in polyethylene glycol, propylene glycol, glycerin, liquid silicone, and/or alcohol). In another embodiment, the galvanic particulates are embedded into an anhydrous carrier (e.g., inside a polymer). In yet another embodiment, the galvanic particulates are encapsulated in compositions of microcapsules, liposomes, or micelles, or embedded in the lipophilic phase of oil-in-water (O/W) or water-in-oil (W/O) types of emulsion systems (e.g., W/O lotion, W/O ointment, or O/W creams), as well as self-emulsifying compositions, in order to achieve self-life stability, retard the activation of the galvanic particulates, or prolong the action of galvanic particulates.

The lip composition preferably comprises, in addition to the galvanic particulates, oils, waxes, emollients, and pigments, which apply color and texture to the lips.

In particular, the lip composition preferably includes at least one structuring agent selected from thickeners, fatty substances, and waxes of the type generally used in personal care or cosmetic compositions. The structuring agent imparts a self-sustaining shape to the lip composition at room temperature. The amount of a structuring agent is preferably from 5% to 90%, or 10% to 30%, most preferably from 10% to 20% of the lip composition.

It is possible to use the materials suitable for use as structuring agents as viscosity modifiers in the case of lip glosses, which generally do not have a self-sustaining shape. When used as viscosity modifiers rather than structuring agents, the amounts of these ingredients used are preferably insufficient to provide a self-sustaining shape at room temperature (18-25° C).

Thickeners may include clays, organoclays, silicas, cellulose derivatives hectorites, synthetic polymers such as an acrylic polymer or an associative polymer of the polyurethane type, and gums, in particular xanthan gum.

Representative fatty substances include silicones in esterified or unesterified liquid form or in esterified solid form, such as behenate dimethicone, polyamide resins, nonsilicone fatty substances, such as oils, pastes and vegetable, mineral, animal and/or synthetic waxes.

Waxes may be used to form a non-transparent composition. As used herein, a "wax" may be any lipophilic fatty compound which is soluble in the liquid fatty phase. The wax, for example, may have a melting point greater than about 45° C, such as, for example greater than about 55° C.

Non-limiting examples of suitable waxes include waxes of natural origin, such as beeswax, carnauba wax, candelilla wax, ouricury wax, Japan wax, cork fiber wax, sugar cane wax, paraffin waxes, lignite wax, microcrystalline waxes, lanolin wax, montan wax and ozokerites, hydrogenated oils such as hydrogenated jojoba oil, jojoba esters, waxes of synthetic origin, such as polyethylene waxes derived from polymerization of ethylene, waxes obtained by Fischer-Tropsch synthesis, fatty acid esters and glycerides, castor oil, and silicone waxes such as derivatives of poly(di)methylsiloxane.

The lip composition may comprise one or more pigments as known in the cosmetic art, including inorganic pigments, lakes, micas and effect pigments. Examples of typical pigments include bromo acid, D&C Red No. 21, D&C Red No. 27 and lakes such as D&C Red No. 34, Calcium lake, and D&C Orange No. 17. Pink shades are made by mixing titanium dioxide with various shades of red. Preferably, the pigments are insoluble in water, so the color will be maintained on the lips over time.

In one embodiment, only the bulk color of the lip composition is adjusted using a pigment, with no color change to the lips on application.

In another embodiment, the pigment imparts a sheer color coverage to lips.

In another embodiment, the pigment imparts a high color change to the lips on application.

The lip composition may take a variety of forms, as known in the cosmetic art, including frosted, matte, sheer, stain, and long-lasting lip color products. Frosted lipsticks, for example may include a pearlizing agent that adds luster to the color. For example, bismuth oxychloride, which is synthetic pearl, imparts a frost or shine. Bismuth subcarbonate may be used as a skin protectant.

Matte lipsticks contain greater amounts of structuring agents and pigment but less emollients. They have more texture than shine. Crème lipsticks are a balance of shine and texture. Lip glosses have a high shine and low color. Lip sheers and stains contain a high level of oil and a medium amount of wax. Lip shimmers typically contain mica or silica particles. Long-lasting color lipsticks often contain silicone oil.

In one embodiment, the lip composition further comprises a liquid crystal. Examples of liquid crystals include ISP Colorflow liquid crystals commercially available from International Specialty Products. Liquid crystals may provide unique color effects to the lip composition, as well as moisturizing benefits.

The lip composition may also comprise preservatives and antioxidants as known in the cosmetic art.

In one embodiment, the lip composition is a lip gloss. The lip gloss prefereably comprises a shine-enhancing film former comprising between about 0.5 and about 80% w/w, preferably between about 0.5 and about 60% w/w, of at least one low molecular weight non-polar, thermoplastic polyolefin shine-enhancing polymer dissolved, dispersed, suspended, or emulsified in an organic solvent. Also included is at least one viscosity increasing agent.

The lip composition may comprise other additives as known in the cosmetic art. For example, hydrophobic conditioning agents may be included in the lip composition. They may be selected from mineral oil, lecithin, hydrogenated lecithin, lanolin, lanolin derivatives, C7-C40 branched chain hydrocarbons, C1-C30 alcohol esters of C1-C30 carboxylic acids, C1-C30 alcohol esters of C2-C30 dicarboxylic acids, monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, propylene glycol diesters of C1-C30 carboxylic acids, C1-C30 carboxylic acid monoesters and polyesters of sugars, polydialkylsiloxanes, polydiarylsiloxanes, polyalkarylsiloxanes, cyclomethicones having 3 to 9 silicon atoms, vegetable oils, hydrogenated vegetable oils, polypropylene glycol C4-C20 alkyl ethers, di C8-C30 alkyl ethers, and combinations thereof.

Emulsifiers may be used in the lip composition, especially in the presence of hydrophilic components. The amount of emulsifier may range from about 0.1 to about 10%, preferably from about 0.3 to about 5%, by weight of the lip composition. Particularly useful are phospholipids such as lecithin and also glycerol fatty acid esters such as glycerol monostearate,

The lip composition may contain polymers or copolymers, as known in the cosmetic art, to add structure the composition. Levels of these materials may range from about 0.1 to about 10%, preferably from about 0.5 to about 8%, more preferably from about 1 to about 5% by weight of the lip composition. Examples include polyvinyl pyrrolidone (PVP) polymers and copolymers, vinyl pyrrolidone/Eicosene copolymer, and vinyl pyrrolidone/hexadecene, commercially available as Ganex 220 and Ganex 216, respectively, from International Specialty Products.

The lip compositions may comprise vitamin compounds, precursors, and derivatives thereof. Vitamin compounds may be in either natural or synthetic form. Suitable vitamin compounds include, but are not limited to, Vitamin A (e.g. beta carotene, retinoic acid, retinol, retinoids, retinyl palmitate, retinyl propionate), Vitamin B (e.g. niacin, niacinamide, riboflavin, pantothenic acid), Vitamin C (e.g. ascorbic acid), Vitamin D (e.g. ergosterol, ergocalciferol, cholecalciferol), Vitamin E (e.g. tocopherol, tocopherol acetate), and Vitamin K (e.g., phytonadione, menadione, phthiocol), compounds. The amount of vitamin used may range from 0.000001 to 2% by weight of the lip composition.

Organic or inorganic sunscreens may be incorporated into the lip compositions. Levels of sunscreen may range from about 0.1 to about 20%, preferably from about 1 to about 5% by weight of the lip composition. Examples of sunscreens include 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyidibenzoylmethane, 4-isopropyl dibenzoylmethane, 3-benzylidene camphor, 3-(4-methylbenzylidene)camphor, titanium dioxide, zinc oxide, silica, iron oxide, and mixtures thereof.

The lip composition may comprise an active agent. As used herein, "active agent" means a compound (e.g., synthetic or natural) that provides a cosmetic or therapeutic effect on the lips or the surrounding tissues, such as a therapeutic drug or cosmetic agent. Examples of therapeutic drugs include small molecules, peptides, proteins, nucleic acid materials, and nutrients such as minerals and extracts. The amount of the active agent in the lip composition will depend on the active agent, other ingredients present in the lip composition, and the desired benefits of the lip composition. In one embodiment, the lip composition contains a safe and effective amount of the active agent, for example, from about 0.001 percent to about 20 percent, by weight, such as from about 0.01 percent to about 10 percent, by weight, of the composition.

The galvanic particulates can be combined with an active agent (such as antimicrobial agents, anti-inflammatory agents, and analgesic agents) to enhance or potentiate the biological or therapeutic effects of that active agent. The galvanic particulates can also be combined with other substances to enhance or potentiate the activity of the galvanic particulates. Substances that can enhance or potentiate the activity of the galvanic particulates include, but are not limited to, organic solvents (such as alcohols, glycols, glycerin, polyethylene glycols and polypropylene glycol), surface active agents (such as nonionic surfactants, zwitterionic surfactants, anionic surfactants, cationic surfactants and polymeric surfactants), and water-soluble polymers. For example, the galvanic particulates can form conjugates or composites with synthetic or natural polymers including by not limited to proteins, polysaccharides, hyaluronic acid of various molecular weight, hyaluronic acid ' analogs, polypeptides, and polyethylene glycols.

The lip composition may contain a chelator or chelating agent. Examples of chelators include, but are not limited to, amino acids such as glycine, lactoferrin, edetate, citrate, pentetate, tromethamine, sorbate, ascorbate, deferoxamine, derivatives thereof, and mixtures thereof. Other examples of chelators useful are disclosed in U.S. Pat. No. 5,487,884 and PCT Publication Nos. 91/16035 and 91/16034.

The lip composition may contain an anti-aging agent. Examples of suitable anti-aging agents include, but are not limited to: inorganic sunscreens such as titanium dioxide and zinc oxide; organic sunscreens such as octyl-methoxy cinnamates; retinoids; dimethylaminoathanol (DMAE), copper containing peptides, vitamins such as vitamin E, vitamin A, vitamin C, and vitamin B and vitamin salts or derivatives such as ascorbic acid di-glucoside and vitamin E acetate or palmitate; alpha hydroxy acids and their precursors such as glycolic acid, citric acid, lactic acid, malic acid, mandelic acid, ascorbic acid, alpha-hydroxybutyric acid, alpha- hydroxyisobutyric acid, alpha-hydroxyisocaproic acid, atrrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, isopropyl pyruvate, methyl pyruvate, mucic acid, pyruvic acid, saccharic acid, saccaric acid 1,4-lactone, tartaric acid, and tartronic acid; beta hydroxy acids such as beta-hydroxybutyric acid, beta-phenyl-lactic acid, and beta-phenylpyruvic acid; tetrahydroxypropyl ethylenediamine, N,N,N',N'-Tetrakis(2-hydroxypropyl)ethylenediamine (THPED); and botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, and safflower; and salts and prodrugs thereof.

The lip composition may contain a plant extract as an active agent. Examples of plant extracts include, but are not limited to, feverfew, soy, glycine soja, oatmeal, what, aloe vera, cranberry, witch-hazel, alnus, arnica, artemisia capillaris, asiasarum root, birch, calendula, chamomile, cnidium, comfrey, fennel, galla rhois, hawthorn, houttuynia, hypericum, jujube, kiwi, licorice, magnolia, olive, peppermint, philodendron, salvia, sasa albo- marginata, natural isoflavonoids, soy isoflavones, and natural essential oils.

The lip composition may contain a buffering agent such as citrate buffer, phosphate buffer, lactate buffer, gluconate buffer, or gelling agents, thickeners, or polymers.

The lip composition or product may contain a fragrance.

The lip composition may comprise an active agent for treating oral herpes, cold sores, canker sores, or treating microbial infections, or improving oral hygiene.

The lip composition may comprise an antibiotic. Examples of antibiotics (or antiseptics) include but are not limited to mupirocin, neomycin sulfate bacitracin, polymyxin B, 1-ofloxacin, tetracyclines (chlortetracycline hydrochloride, oxytetracycline - 10 hydrochloride and tetrachcycline hydrochoride), clindamycin phsphate, gentamicin sulfate, metronidazole, hexylresorcinol, methylbenzethonium chloride, phenol, quaternary ammonium compounds, tea tree oil, and their pharmaceutically acceptable salts and prodrugs.

The lip composition may comprise an antimicrobial. Examples of antimicrobials include but are not limited to salts of chlorhexidine, such as lodopropynyl butylcarbamate, diazolidinyl urea, chlorhexidene digluconate, chlorhexidene acetate, chlorhexidene isethionate, and chlorhexidene hydrochloride. Other cationic antimicrobials may also be used, such as benzalkonium chloride, benzethonium chloride, triclocarbon, polyhexamethylene biguanide, cetylpyridium chloride, methyl and benzothonium chloride. Other antimicrobials include, but are not limited to: halogenated phenolic compounds, such as 2,4,4',-trichloro-2- hydroxy diphenyl ether (Triclosan); parachlorometa xylenol (PCMX); and short chain alcohols, such as ethanol, propanol, and the like. In one embodiment, the alcohol is at a low concentration (e.g., less than about 10% by weight of the carrier, such as less than 5% by weight of the carrier) so that it does not cause undue drying of the barrier membrane.

The lip composition may comprise an anti-viral agent. Examples of anti-viral agents for viral infections such as herpes and hepatitis, include, but are not limited to, imiquimod and its derivatives, podofilox, podophyllin, interferon alpha, acyclovir, famcyclovir, valcyclovir, reticulos and cidofovir, and salts and prodrugs thereof.

The lip composition may comprise an anti-inflammatory agent. Examples of anti-inflammatory agent, include, but are not limited to, suitable steroidal anti-inflammatory agents such as corticosteroids such as hydrocortisone, hydroxyltriamcinolone alphamethyl dexamethasone, dexamethasone-phosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, fluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, triamcinolone, and salts are prodrugs thereof. In one embodiment, the steroidal anti-inflammatory for use in the present invention is hydrocortisone. A second class of anti-inflammatory agents which is useful in the compositions of the present invention includes the nonsteroidal anti-inflammatory agents.

The lip composition may be made using conventional methods. A mixture of pigments is finely ground, and heated structuring agents are added thereto. Oils and emollients may be added for specific requirements. The resulting, hot liquid mixture is then poured into cold metal molds where it solidifies and is further chilled. The formed lip composition may be put briefly heated (half a second) to remove surface imperfections.

The galvanic particulates are preferably ground before adding them to the other ingredients of the lip composition. This reduces their particle size and reduces aggregation of the galvanic particulates.

According to the invention, a lip composition comprising galvanic particulates provides a variety of unexpected lip benefits, including enhanced lip color, reduced fine lines and wrinkles, increased fullness, improved moisturization, smoothness, texture, and improved definition and lip contour.

The invention provides a method of increasing oxy-hemoglobin levels in lips by at least 10 percent, preferably at least 25 percent, by topically applying to the lips a lip composition comprising galvanic particulates. Applicants have found that oxy-hemoglobin levels in the lips increase after topical application of the present lip composition, for example two, preferably three times a day for two, four, or eight weeks.

The following non-limiting examples further illustrate the invention.

### Example 1

0.1 % copper coated zinc galvanic particulates were manufactured by electroless plating of copper onto zinc powder as follows. 10g of ≤45-micron zinc powder was spread evenly onto a vacuum filter buchner funnel with a 0.22 micron filter. 5g of copper acetate solution was then poured evenly onto the zinc powder, and allowed to react for approximately 30 seconds. A suction was then applied to the filter until the filtrate was completely suctioned out. The resulting powder cake was then loosened, and 10 g of deionized water was added and then suctioned off. 10g of ethanol was then added to the powder under suction. The powder was then carefully removed from the filter system and allowed to dry in a desiccator.

### Example 2

A small-scale, randomized, double-blind, placebo- and benchmark-controlled clinical study was conducted to evaluate the effect of compositions containing galvanic particulates on lip-related benefits such as anti-aging, lip beauty, and lip health. The study population consisted of females ages 40-65, Fitzpatrick Skin Type 1-4 who at the time of enrollment (baseline), experienced the following based on expert grade and self report: moderate to severe lines around lip contour, fine vertical lines on lips, lips that have lost color/look paler, lip edges that are less defined, lips that are thinner/less full, lips that experience lipstick bleeds and mild to moderate dry lips.

Four lip compositions in the form of lip balms/sticks having a common base formulation (Comparative Product A, Table 1) were used in the study. Two contained galvanic particulates (Products 1 and 2) and two did not (Comparative Products A and B). There were n=11 subjects per lip composition, and the subjects used the products three times per day: in the morning, afternoon (after lunch meal), and in the evening.

The formulations for the lip compositions are shown in Tables 1-4.

**TABLE 1: Comparative Product A**

| **CHEMICAL NAME (CTFA)** | **% (w/w)** |
|---|---|
| Polybutene | 17.35% |
| Phenyltrimethicone | 10.00% |
| Caprylic/ Capric Triglyceride | 3.00% |
| Octyldodecanol | 5.00% |
| Stearoyl inulin | 1.00% |
| Petrolatum | 11.350% |
| Butyrospermum Parkeii (Shea Butter) | 2.000% |
| Astrocaryum Muru-Muru Butter | 3.000% |
| Ozokerite | 7.000% |
| Pentaerythrityl Distearate | 2.000% |
| Carnauba (Copernicia Cerifera) Wax | 1.000% |
| Euphorbia (Candelilla) Cerifera Wax | 4.00% |
| Polyethylene | 4.00% |
| Hydrogenated Lanolin | 5.00% |
| Polyester-4 | 6.00% |
| Octinoxate | 7.50% |
| Titanium Dioxide and Hydrogenated Polyisobutene | 7.00% |
| C10-30 Cholesterol/ Lanosterol Esters | 3.00% |
| Pentaerythrityl Di-t-butyl-Hydroxyhydrocinnamate | 0.10% |
| Methylparaben | 0.10% |
| Tocopherol | 0.50% |
| Castor Oil & Sodium Saccharin & BHT | 0.100% |
| TOTAL | 100.00% |

**TABLE 2: Product 1**

| **CHEMICAL NAME (CTFA)** | **% (w/w)** |
|---|---|
| Polybutene | 17.35% |
| Phenyltrimethicone | 9.00% |
| Caprylic/ Capric Triglyceride | 3.00% |
| Octyldodecanol | 5.00% |
| Stearoyl inulin | 1.00% |
| Petrolatum | 11.350% |
| Butyrospermum Parkeii (Shea Butter) | 2.000% |
| Astrocaryum Muru-Muru Butter | 3.000% |
| Ozokerite | 7.000% |
| Pentaerythrityl Distearate | 2.000% |
| Carnauba (Copernicia Cerifera) Wax | 1.000% |
| Euphorbia (Candelilla) Cerifera Wax | 4.00% |
| Polyethylene | 4.00% |
| Hydrogenated Lanolin | 5.00% |
| Polyester-4 | 6.00% |
| Octinoxate | 7.50% |
| Titanium Dioxide and Hydrogenated Polyisobutene | 7.00% |
| C10-30 Cholesterol/ Lanosterol Esters | 3.00% |
| Pentaerythrityl Di-t-butyl-Hydroxyhydrocinnamate | 0.10% |
| Methylparaben | 0.10% |
| Tocopherol | 0.50% |
| Castor Oil & Sodium Saccharin & BHT | 0.100% |
| Example 1 Galvanic Particulates | 1.000% |
| TOTAL | 100.00% |

**TABLE 3: Comparative Product B**

| **CHEMICAL NAME (CTFA)** | **% (w/w)** |
|---|---|
| Polybutene | 17.35% |
| Phenyltrimethicone | 9.00% |
| Caprylic/ Capric Triglyceride | 3.00% |
| Octyldodecanol | 5.00% |
| Stearoyl inulin | 1.00% |
| Petrolatum | 11.350% |
| Butyrospermum Parkeii (Shea Butter) | 2.000% |
| Astrocaryum Muru-Muru Butter | 3.000% |
| Ozokerite | 7.000% |
| Pentaerythrityl Distearate | 2.000% |
| Carnauba (Copernicia Cerifera) Wax | 1.000% |
| Euphorbia (Candelilla) Cerifera Wax | 4.00% |
| Polyethylene | 4.00% |
| Hydrogenated Lanolin | 5.00% |
| Polyester-4 | 6.00% |
| Octinoxate | 7.50% |
| Titanium Dioxide and Hydrogenated Polyisobutene | 7.00% |
| C10-30 Cholesterol/ Lanosterol Esters | 3.00% |
| Pentaerythrityl Di-t-butyl-Hydroxyhydrocinnamate | 0.10% |
| Methylparaben | 0.10% |
| Ethylhexyl Palmitate & Tribehenin & sorbitan Isostearate & Palmitoyl Oligopeptide | 1.00% |
| Tocopherol | 0.50% |
| Castor Oil & Sodium Saccharin & BHT | 0.100% |
| TOTAL | 100.00% |

**TABLE 4: Product 2**

| **CHEMICAL NAME (CTFA)** | **% (w/w)** |
|---|---|
| Polybutene | 17.35% |
| Phenyltrimethicone | 8.00% |
| Caprylic/ Capric Triglyceride | 3.00% |
| Octyldodecanol | 5.00% |
| Stearoyl inulin | 1.00% |
| Petrolatum | 11.350% |
| Butyrospermum Parkeii (Shea Butter) | 2.000% |
| Astrocaryum Muru-Muru Butter | 3.000% |
| Ozokerite | 7.000% |
| Pentaerythrityl Distearate | 2.000% |
| Carnauba (Copernicia Cerifera) Wax | 1.000% |
| Euphorbia (Candelilla) Cerifera Wax | 4.00% |
| Polyethylene | 4.00% |
| Hydrogenated Lanolin | 5.00% |
| Polyester-4 | 6.00% |
| Octinoxate | 7.50% |
| Titanium Dioxide and Hydrogenated Polyisobutene | 7.00% |
| C10-30 Cholesterol/ Lanosterol Esters | 3.00% |
| Pentaerythrityl Di-t-butyl-Hydroxyhydrocinnamate | 0.10% |
| Methylparaben | 0.10% |
| Ethylhexyl Palmitate & Tribehenin & sorbitan Isostearate& Palmitoyl Oligopeptide | 1.00% |
| Tocopherol | 0.50% |
| Castor Oil & Sodium Saccharin & BHT | 0.100% |
| Example 1 Galvanic Particulates | 1.000% |
| TOTAL | 100.00% |

The subjects were evaluated at Baseline, Week 1 and Week 8. At each of those time points subject self-assessment questionnaires were completed, high resolution digital images of the lips were taken, and spectral imaging of the lips was conducted.

The high resolution digital images were graded by an expert grader (blinded to the treatment groups) to provide an objective assessment of changes in the lips. The expert grader used eight visual grading parameters to grade the digital images:
1) lines on the lips,
2) lines around the lips,
3) texture,
4) even tone,
5) color,
6) fullness,
7) definition, and
8) dryness (visual assessment only).

The expert grading results indicated that the Product 1 lip composition was significantly better (p<0.05) versus the Comparative Product A in improving lines around the lips and directionally better (p<0.10) in definition and lines on lips at week 8. Moreover, Product 1 was the only lip composition that provided at least directionally significant (p<0.10) improvement versus baseline in all eight of the grading parameters scored at week 8.

The Product 2 lip composition outperformed Comparative Product B by showing improvement versus baseline in fullness of lips (p<0.05), even tone (p<0.05) and lines on lips (p<0.1), whereas Comparative Product B did not.

Overall, the digital images demonstrated visible lip improvements by treating with both Product 1 and Product 2 in lip color, fine lines, and fullness in just one week, with continuing efficacy in all parameters at week 8.

Oxy-hemoglobin analysis was performed on the processible spectral images (n=7-9 subjects per treatment group). As shown in Figure 1, the Product 1 lip composition showed an almost 25% increase (p=0.05) in oxy-hemoglobin levels at week 8 versus baseline levels. Comparative Product A did not show a significant change. This increase in oxy-hemoglobin level is believed to have contributed to the observed visible improvement in lip color demonstrated in the self-assessment and expert grading data.

The results of the subject self-assessment questionnaires indicated that Product 2 consistently ranked higher than all other products, starting at Week 1. Product 2 also had the most between-treatment significance and was the only lip composition that showed significant (p<0.05) improvement in the self-assessments versus baseline in all lip parameters at week 8.

In addition, the percentage of top-two-box scores for Product 2 was higher than those of the other lip compositions for the following parameters: "lips have a healthy glow" and "feel more radiant," "overall lips look and feel better," "more youthful," "fuller/more supple" and "delivered better results than other products she normally uses."

Overall, the lip compositions containing the galvanic particulates demonstrated statistically significant (p<0.05) improvements versus baseline and versus placebo and a similar composition not containing galvanic particulates in lip properties as early as the first evaluation (week 1) in subject self-assessments and expert grading of clinical images. The lip compositions according to the invention also continued to provide improvements at the final evaluation. The improvements observed included: enhanced lip color, reduced fine lines and wrinkles, increased fullness, improved moisturization, smoothness, texture, and improved definition and lip contour.

### Example 3-5

The following lip compositions according to the invention were made using the ingredients shown in Table 5 (amounts in weight percent). The compositions were made by heating Phase A to 65° C, then adding Phase B thereto and mixing until homogenous. The resulting mixtures were then cooled down while stirring.

**TABLE 5**

| | EXAMPLE | | |
|---|---|---|---|
| **PHASE A** | **3** | **4** | **5** |
| Hydrogenated Polyisobutene (Panalane L -14E) | 10 | 7 | 9 |
| Hydrogenated Polyisobutene (Panalane H 300E) | 10 | 15 | 10 |
| Polybutene | 9 | 5 | 6 |
| Phenyltrimethicone | 4.1 | 5 | 4 |
| Thixogel 1538 | 25 | 22 | 30 |
| Myristyl Myristate | 3 | 3 | 3 |
| Isododecane | 10 | 9.9 | 5.9 |
| Bis-Behenyl/Isostearyl/ Phytosteryl Dimer Dilinoleyl Dimer Dilinoleate | 4 | 5 | 5 |
| Giovarez Ac 5099 | 5 | 11 | 10 |
| Hydrogenated Lanolin (Lipolan-Distilled) | 4 | 4 | 4 |
| Jeesilc ps-VHLV | 10 | 7 | 7.5 |
| Butyrospermum Parkeii | 2 | 3.95 | 2.95 |
| | | | |

| **PHASE B** | | | |
|---|---|---|---|
| Red 6 | 0.6 | 0.05 | 0.05 |
| Chromalite Magenta | 1 | 0 | 0 |
| Zinc & Copper Galvanic Particulates | 1.2 | 1 | 1.5 |
| Methylparaben | 0.1 | 0.1 | 0.1 |
| Ethylhexyl Palmitate & Tribehenin & sorbitan Isostearate& Palmitoyl Oligopeptide | 1 | 1 | 1 |
| **TOTAL** | **100** | **100** | **100** |

### Examples 6-13

The following lip compositions according to the invention were made using the ingredients shown in Table 6 (amounts in weight percent). The compositions were each made by mixing Phase A and grinding it using roller mill. Phase B was added to Phase A and the mixture was heated to 75-80° C until the waxes melted. Phase C was then added thereto and the ingredients were mixed until homogenous. Phase D was separately ground through a tripler roller mill and then added to the bulk mixture at 70° C. The composition was then poured, while mixing, into a mold at 60° C.

**TABLE 6**

| **PHASE A** | **6** | **7** | **8** | **9** | **10** | **11** | **12** | **13** |
|---|---|---|---|---|---|---|---|---|
| | | | | | | | | |
| Micro titanium dioxide | 1.1 | 1.1 | 1.1 | 1 | 1 | 1.1 | 1.1 | 1.1 |
| Titanium Dioxide (and) Triethoxycaprylylsilane | 0.16 | 0.36 | 1 | 0.16 | 2 | 4 | 5 | 5 |
| Petrolatum | 9.1 | 9.1 | 8.6 | 6.8 | 7.5 | 7.5 | 7 | 7.5 |
| Octinoxate | 7.5 | 7 | 5 | 6.5 | 7.4 8 | 7.5 | 7.5 | 7.5 |
| Oxybenzone (Neoheliophan) | 5 | 5 | 3 | 5 | 5 | 5 | 5 | 5 |
| Hydrogenated Polyisobutene | 8 | 10 | 9 | 8 | 8 | 8 | 8 | 8 |
| Red 7 | 0.12 | 0.15 | 0.12 | 0.12 | 0.5 | 0.6 | 1 | 1 |
| Red 6 | 0.04 | 0.08 | 0.04 | 0.06 | 0.2 | 0.3 | 0.2 | 0.25 |
| Iron Oxide (and) Triethoxycaprylylsilane | 0.33 | 4.33 | 0.33 | 0.45 | 2 | 1 | 3 | 3 |
| Iron Oxide (and) Triethoxycaprylylsilane | 0.08 | 0.08 | 0.01 | 0.09 | 0.0 2 | 0.1 | 0.2 | 0.05 |
| | | | | | | | | |

| **PHASE B** | | | | | | | | |
|---|---|---|---|---|---|---|---|---|
| Castor Oil | 0 | 2 | 5 | 8.5 | 5 | 7 | 8 | 10.5 |
| Hydrogenated Lanolin | 7 | 6 | 5 | 8 | 4.5 | 3 | 4 | 3.3 |
| Hydrogenated Polyisobutene | 9 | 8 | 5 | 7.5 | 6 | 4 | 4.2 | 3 |
| Polybutene | 8 | 7 | 9 | 6.5 | 6 | 6 | 6 | 6 |
| Phenyltrimethicone | 7 | 7 | 3 | 3 | 6.9 | 5.4 | 2 | 3 |
| Alkyl Silicone Wax | 1 | 1 | 2 | 1 | 1.2 | 1.2 | 1.2 | 1 |
| Cetyl Lactate | 3 | 3 | 3 | 3.1 | 2.3 | 2.9 | 2.9 | 1 |
| Myristyl Myristate | 3.4 | 3.4 | 3.4 | 3 | 2 | 3.4 | 3 | 2 |
| Bis-Behenyl/Isostearyl/Phytost eryl Dimer Dilinoleyl Dimer Dilinoleate | 3 | 3 | 4 | 2.5 | 2.9 | 2.9 | 2.9 | 3 |
| | | | | | | | | |
| Butyrospermum Parkeii | 3 | 2.9 | 2.9 | 3 | 3.3 | 3.3 | 3 | 3 |
| Ozokerite | 6 | 5.8 | 7 | 6.6 | 6.1 | 6.1 | 6.1 | 6.1 |
| Carnauba (Copernicia Cerifera) Wax | 1 | 1.2 | 4 | 0.8 | 1 | 1 | 1 | 1 |
| Euphorbia (Candelilla) Cerifera Wax | 3 | 3.2 | 4 | 2.9 | 3.2 | 3.2 | 3.2 | 3.2 |
| Polyethylene | 3.37 | 3.5 | 3.3 | 4 | 5 | 5 | 4 | 5 |
| | | | | | | | | |

| **PHASE C** | | | | | | | | |
|---|---|---|---|---|---|---|---|---|
| Silica (VM 2270, Kobo) | 0.1 | 0.2 | 0.2 | 0.4 | 0.2 | 0.2 | 0.2 | 0.2 |
| Silica (MS 500/3H, Kobo) | 1.2 | 2 | 1.2 | 0.52 | 1.2 | 1.2 | 1.2 | 1.2 |
| Mica | 2.4 | 0.5 | 2.2 | 2.4 | 2.8 | 2.4 | 2.4 | 2.4 |
| Ethylhexyl Palmitate & Tribehenin & sorbitan Isostearate& Palmitoyl Oligopeptide | 1.1 | 1.1 | 1.1 | 1.1 | 1.2 | 1.2 | 1.2 | 1.2 |
| | | | | | | | | |

| **PHASE D** | | | | | | | | |
|---|---|---|---|---|---|---|---|---|
| Zinc & Copper Galvanic | | | | | | | | |
| Parcticulates | 1 | 1 | 1.5 | 2 | 0.5 | 0.5 | 0.5 | 0.5 |
| Petrolatum | 5 | 5 | 5 | 5 | 5 | 5 | 5 | 5 |
| | | | | | | | | |
| **TOTAL** | **100** | **100** | **100** | **100** | **100** | **100** | **100** | **100** |

## Claims

1. A lip composition comprising: a) at least one structuring agent selected from thickeners, fatty substances, and waxes, and b) galvanic particulates comprising a first conductive material and a second conductive material, wherein both said first conductive material and said second conductive material are exposed on the surface of said galvanic particulates, the particle size of said galvanic particulates is from about 10 nanometers to about 100 micrometers, and the difference in Standard Potentials of the first conductive material and the second conductive material is at least about 0.2 V.

2. The lip composition of claim 1, wherein said galvanic particulates comprise said first conductive material partially coated with said second conductive material.

3. The lip composition of claim 1 or claim 2, wherein said galvanic particulate comprises at least 95 percent, by weight, of said first conductive material and said second conductive material.

4. The lip composition of any preceding claim, wherein said first conductive material is zinc.

5. The lip composition of any preceding claim, wherein said second conductive material is copper or silver.

6. The lip composition of any preceding claim, where said structuring agent comprises a wax.

7. The lip composition of any preceding claim further comprising a liquid crystal.

8. The lip composition of any preceding claim, wherein said lip composition increases the oxy-hemoglobin levels in the lips by at least 10 percent after topical application to the lips three times per day for eight weeks.

9. A method of increasing oxy-hemoglobin levels in lips by at least 10 percent, comprising topically applying to the lips a lip composition comprising galvanic particulates comprising a first conductive material and a second conductive material, wherein both said first conductive material and said second conductive material are exposed on the surface of said galvanic particulates, the particle size of said galvanic particulates is from about 10 nanometers to about 100 micrometers, and the difference in Standard Potentials of the first conductive material and the second conductive material is at least about 0.2 V.

10. A method of improving the condition or appearance of lips selected from enhancing lip color, reducing fine lines and wrinkles, increasing fullness, improving moisturization, smoothness, or texture, and improving definition and lip contour, comprising topically applying to the lips a lip composition comprising galvanic particulates comprising a first conductive material and a second conductive material, wherein both said first conductive material and said second conductive materials are exposed on the surface of said galvanic particulates, the particle size of said galvanic particulates is from about 10 nanometers to about 100 micrometers, and the difference in Standard Potentials of the first conductive material and the second conductive material is at least about 0.2V.
